# EUROPEAN PATENT APPLICATION

(11) **EP 4 147 657 A1**
(43) Date of publication of application: **15.03.2023**
(21) Application number: 20934761.6
(22) Date of filing: 12.06.2020
(51) Int. Cl.: A61B 18/14

(54) **ENDOSCOPIC SURGICAL ELECTRODE ASSEMBLY**

(30) Priority: 06.05.2020 CN 202010370807
(71) Applicant: Anjin Medical Technology (Beijing) Co., Ltd., Beijing 100095 (CN)
(72) Inventor: NAKAHIRA, Isamu, Beijing 100102 (CN); GUO, Zhigang, Beijing 100095 (CN); QIAO, Jianjiang, Beijing 100095 (CN); LU, Yuchuan, Beijing 100095 (CN)
(74) Representative: Dehns
(86) International application number: PCT/CN2020/095740
(87) International publication number: WO 2021/223288

(57) **Abstract**

An endoscopic surgical electrode assembly, comprising: an electrode core wire (1), an extension/retraction control member (2), and an insulating outer sleeve (3). The electrode core wire (1) comprises: an electrode head (101), a coaxial cable (102), and an electrode insulating tube (103). The electrode head (101) is electrically connected to an output end of an external host by means of the coaxial cable (102). The electrode insulating tube (103) is sleeved on the electrode head (101). The insulating outer sleeve (3) is movably sleeved on the exterior of the electrode core wire (1), and the end where the electrode head (101) is located is a preset distance longer than the electrode insulating tube (103). The extension/retraction control member (2) is used for controlling the insulating outer sleeve (3) to move, such that the electrode head (101) extends out or retracts into the insulating outer sleeve (3). The electrode assembly can avoid high-frequency current leakage in an operation process and influence thereof on the concentration of electric field energy on the electrode head (101), and avoid the electrode head (101) from scratching an endoscopic channel when passing therethrough and influence of the scratching on the service life of an endoscopy.

## Description

The present application claims priority to Chinese Patent Application No. 202010370807.X, filed on May 06, 2020, and entitled "Electrode Assembly for Endoscopic Surgery", the entire contents of which are hereby incorporated by reference in its entirety.

### TECHNICAL FIELD

The present disclosure relates to the field of medical devices, and in particular to an electrode assembly for endoscopic surgery.

### BACKGROUND

With the advancement of the medical imaging technology and the development of the minimally invasive surgery, endoscopic surgery is widely used in the fields of thoracic cavity, abdominal cavity, nasal cavity, gynecology and urology. Compared with open surgery, endoscopic surgery can reduce iatrogenic injury, maximally protect and restore the function of the surgical site, and improve the quality of life of patients after their illness. In the case of intracranial endoscopic surgery, for example, generally a small hole is opened in the skull, into which an endoscope is inserted, and an elongated endoscopic electrode is used to enter the brain through an endoscope channel. The camera lens on the endoscope is used to observe and determine the state of the lesion area while controlling the endoscopic electrode to perform surgical operations such as punching, fistulation, coagulation, hemostasis, and incision by high-frequency generator. The endoscopic electrode includes a metal wire disposed in the endoscope channel and a negative electrode plate attached to the patient's body. In use, a high-frequency voltage is applied between the metal wire and the negative electrode plate to coagulate or incise a tissue at a lesion site by the metal wire in contact with the tissue.

However, the endoscopic surgery is generally performed in cerebrospinal fluid or infused saline solution. The exposed metal wire can not make the high-frequency energy focus on the electrode head well in the saline solution, and there is much leakage of high-frequency current, which may easily cause damage to the human body. In addition, there is a large space between the endoscope operation channel and the metal wire, so the metal wire may easily scratch the endoscope channel in the process of passing through the endoscope channel, thereby affecting the service life of the endoscope.

### SUMMARY

Embodiments of the present disclosure provide an electrode assembly for endoscopic surgery, which serves to avoid high frequency current leakage during operation, and prevent the electrode tip from scratching the endoscope channel while passing through the endoscope channel and affecting the service life of the endoscope.

The electrode assembly for endoscopic surgery includes an electrode core wire, a telescopic control member, and an insulating outer sleeve; the electrode core wire includes an electrode tip, a coaxial cable and an electrode insulating tube, wherein the electrode tip is electrically coupled to an output end of an external host through the coaxial cable, and the electrode insulating tube sleeves on the electrode tip; the insulating outer sleeve movably sleeves on the outside of the electrode core wire, and one end of the insulating outer sleeve where the electrode tip is located is a preset distance longer than the electrode insulating tube; the telescopic control member is configured to control the movement of the insulating outer sleeve so that the electrode tip extends out of the insulating outer sleeve or retracts into the insulating outer sleeve.

In the surgical electrode assembly provided by the embodiment of the present disclosure, an electrode tip, a coaxial cable and an electrode insulating tube are provided, the electrode tip is electrically coupled to the output end of the external host through the coaxial cable, and the electrode insulating tube sleeves on the electrode tip, which improves the insulating performance, and avoids high frequency leakage of the electrode tip during the surgery, thus avoiding the weakening of the electric field intensity of the tip, the failure to achieve the field intensity required for coagulation or incision using the near field effect, and the unexpected damage to the human body caused by the high frequency leakage. The telescopic control member and the insulating outer sleeve are provided such that the insulating outer sleeve movably sleeves on the outside of the electrode insulating tube. One end of the insulating outer sleeve where the electrode tip is located is a preset distance longer than the electrode insulating tube, so that the telescopic control member controls the movement of the insulating outer sleeve to retract the electrode tip into the insulating outer sleeve, which ensures that the electrode tip can retract into the insulating outer sleeve during passing through the endoscope channel and can extend out of the insulating outer sleeve after entry into the lesion area to perform the subsequent surgical operations, thereby preventing the electrode tip from scratching the endoscope channel while passing through the endoscope channel, and avoiding affecting the service life of the endoscope.

### BRIEF DESCRIPTION OF THE DRAWINGS

For a clearer illustration of technical features in the embodiments of the present disclosure, a brief description of the drawings for the embodiments will be given below. Obviously, the drawings described below involve only some embodiments of this disclosure. For those of ordinary skill in the art, other drawings can be derived from these drawings without any inventive efforts. In the drawings:
FIG. 1 is a partial sectional view of an electrode tip of an electrode assembly for surgery according to an embodiment of the present disclosure;
FIG. 2 is a diagram showing a first structural example of a telescopic control member according to an embodiment of the present disclosure;
FIG. 3 is a diagram showing a first specific example of a telescopic control member according to an embodiment of the present disclosure;
FIG. 4 is a schematic diagram of a first structure example of a tubular body according to an embodiment of the present disclosure;
FIG. 5 is a schematic diagram of a first structure example of a movable member according to an embodiment of the present disclosure;
FIG. 6 is a schematic diagram showing a second structure example of a telescopic control member according to an embodiment of the present disclosure;
FIG. 7 is a diagram showing a second specific example of a movable member according to an embodiment of the present disclosure;
FIG. 8 is a diagram showing a second specific example of a tubular body according to an embodiment of the present disclosure;
FIG. 9 is a schematic diagram of a second structure example of a fixed cylinder according to an embodiment of the present disclosure.

The reference numerals in the drawings are as follows.
1 electrode core wire;
101 electrode tip;
102 coaxial cable;
1021 center conductor;
1022 insulating layer;
1023 conducting layer;
1024 outer insulating skin;
103 electrode insulating tube;
104 insulating inner sleeve;
105 coaxial connector;
2 telescopic control member;
201 tubular body;
2011 guiding groove;
2012 reed;
202 movable member;
2021 positioning hole;
2022 limiting snap ring;
203 end cap;
204 fixed tube;
205 threaded connecting ring;
206 positioning pin;
207 fixed cylinder;
2071 groove;
3 insulating outer sleeve;
4 sealing member.

### DETAILED DESCRIPTION

For a clearer understanding of the objectives, technical features and effects of the embodiments of the present disclosure, specific embodiments will now be described with reference to the drawings. The described embodiments are intended only to schematically illustrate and explain this invention and do not limit the scope of the present disclosure.

It should be noted that there are generally three endoscope channels, through one of which physiological saline is injected into the lesion area, through another of which a camera is inserted into the skull, and through yet another of which an electrode assembly is inserted into the skull during intracranial endoscopic surgery.

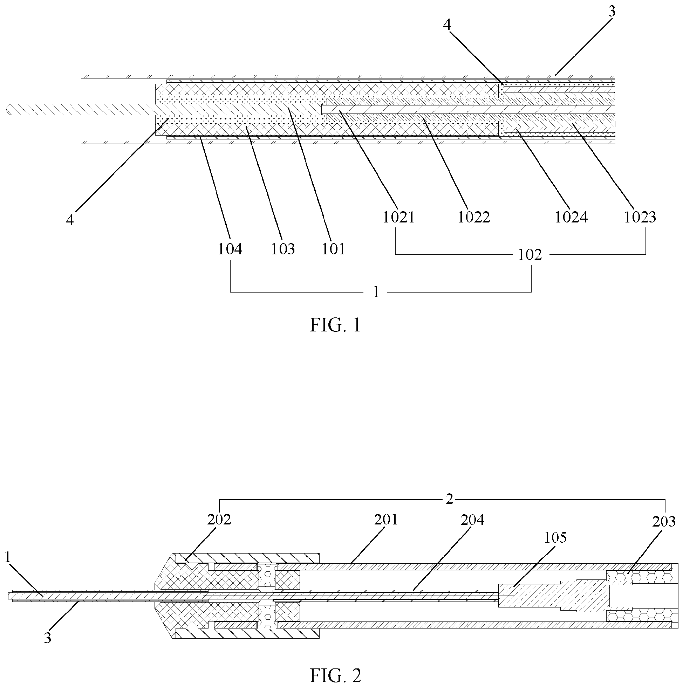

The embodiment of the present invention provides an electrode assembly for endoscopic surgery. As shown in FIGS. 1 and 2, the electrode assembly for endoscopic surgery includes an electrode core wire 1, a telescopic control member 2, and an insulating outer sleeve 3. The electrode core wire 1 is electrically coupled to an output end of an external host through a coaxial cable 102. The electrode insulating tube 103 sleeves on the electrode tip 101. The insulating outer sleeve 3 movably sleeves on the outside of the electrode core wire 1, and one end of the insulating outer sleeve 3 where the electrode tip 101 is located is a preset distance longer than the electrode insulating tube 103. The telescopic control member 2 is configured to control the movement of the insulating outer sleeve 3 so that the electrode tip 101 extends out of the insulating outer sleeve 3 or retracts into the insulating outer sleeve 3.

When an intracranial surgery is required, the telescopic control member 2 is used to control the movement of the insulating outer sleeve 3 to retract the electrode tip 101 into the insulating outer sleeve 3, and the electrode tip 101 is sent to the lesion area in the skull along the endoscopic channel. After the electrode tip 101 reaches the predetermined position, the telescopic control member 2 is used to control the movement of the insulating outer sleeve 3 to extend the electrode tip 101 out of the insulating outer sleeve 3. Then the external host is used to drive the electrode tip 101 through the coaxial cable 102 to perform surgical operations such as punching, incision, coagulation, hemostasis and so on in the intracranial lesion area.

In the electrode assembly for endoscopic surgery provided by the embodiment of the present disclosure, an electrode tip 101, a coaxial cable 102 and an electrode insulating tube 103 are provided, the electrode tip 101 is electrically coupled to the output end of the external host through the coaxial cable 102, and the electrode insulating tube 103 sleeves on the electrode tip 101, which improves the insulating performance, and avoids high frequency leakage of the electrode tip 101 during the surgery, thus avoiding the weakening of the electric field intensity of the tip, the failure to achieve the field intensity required for coagulation or incision using the near field effect, and the damage to the human body caused by the high frequency leakage. The telescopic control member 2 and the insulating outer sleeve 3 are provided such that the insulating outer sleeve 3 movably sleeves on the outside of the electrode insulating tube 103. One end of the insulating outer sleeve 3 where the electrode tip 101 is located is a preset distance longer than the electrode insulating tube 103, so that the telescopic control member 2 controls the movement of the insulating outer sleeve 3 to retract the electrode tip 101 into the electrode insulating tube 3, which ensures that the electrode tip 1 can retract into the insulating outer sleeve 3 during passing through the endoscope channel and can extend out of the insulating outer sleeve 3 after entry into the lesion area to perform the subsequent surgical operations, thereby preventing the electrode tip 101 from scratching the endoscope channel while passing through the endoscope channel, and avoiding affecting the service life of the endoscope.

The electrode insulating tube 103 has excellent electrical insulation strength and erosion resistance, so as to enhance insulation performance between the electrode tip 101 and a conducting layer1023 (negative electrode) as described below, improve the equivalent impedance of the electrode, and prevent ablation of a connection point between the electrode tip 101 and the coaxial cable 102 by electrical discharge when vaporization and cutting actions are performed between the electrode tip 101 and the tissue, thus improving the durability of the whole electrode assembly. For example, the electrode insulating tube 103 may be a ceramic tube. The electrode insulating tube 103 may have an inner diameter of 0.4 mm to 1.2 mm, an outer diameter of 0.8 mm to 2.0 mm, and a length of 4 mm to 12 mm, which are determined based on sizes of the electrode tip and the coaxial cable.

In order to prevent the electrode tip 101 and the conducting layer 1023 (negative electrode) of the coaxial cable 102 from conducting in cerebrospinal fluid or physiological saline through the outside of the electrode insulating tube 103, and to avoid a reduction in insulation, as shown in FIG. 1, the surgical electrode assembly further includes an insulating inner sleeve 104 which seals and sleeves the outside of the electrode insulating tube 103 and the outer insulating skin 1024 of the coaxial cable 102.

The electrode insulating tube 103 may be disposed on the insulating inner sleeve 104 by gluing. The difference between the inner diameter of the electrode insulating tube 103 and the outer diameter of the electrode tip 101 is smaller than a predetermined threshold value greater than 0, so that there is only a small space between the electrode insulating tube 103 and the electrode tip 101, so as to prevent liquid from entering the inside of the insulating inner sleeve 104 during the surgery.

In installation, the electrode tip 101 is connected to the center conductor 1021 of the coaxial cable 102 at an intermediate position of the electrode insulating sleeve 103; a portion of the electrode tip 101 is exposed to the outside of the electrode insulating tube 103; the gap between the electrode insulating tube 103 and the electrode tip 101, the gap between the insulating inner sleeve 104 and the coaxial cable 102, and the gap between the electrode insulating tube 103 and the coaxial cable 102 are filled with the sealing member 4 (see FIG. 1). The sealing member 4 may be silicone rubber, ceramic rubber, or the like with excellent insulating properties. The insulating inner sleeve 104 sleeves on the outside of the electrode insulating tube 103 and the outside of the coaxial cable 102. The electrode insulating sleeve 103 and part of the coaxial cable 102 inserted into the insulating inner sleeve 104 are bonded to the insulating inner sleeve 104 with glue, and the bonding may only include bonding to the rear portion of the electrode insulating sleeve 103, and there is no need to bond the rear long coaxial cable 102 to the insulating inner sleeve 104.

The insulating inner sleeve 104 and the insulating outer sleeve 3 may be smooth sleeves with preset flexibility and rigidity and made up of organic polymer material. In addition to the above properties, the sleeve made up of the organic polymer material also has excellent electrical insulation and biocompatibility. The organic polymer material may be, for example, polyimide. By bonding the electrode insulating tube 103 and part of the coaxial cable 102 inserted into the insulating inner sleeve 104 to the insulating inner sleeve 104, which, on the one hand, may serve as a seal, and prevent cerebrospinal fluid or physiological saline from entering between the electrode tip 101 and the conducting layer 1023 during the surgery and causing a decrease in the equivalent impedance of the electrode, and on the other hand, may improve the rigidity of the coaxial cable 102. Since the coaxial cable 102 has a length of about 400 mm to 900 mm and a diameter of about 0.6 mm to 1.5 mm, and is very flexible, the coaxial cable 102 without being bonded to the insulating inner sleeve 104 may bend and fail to slid due to the high coefficient of friction when someone pushes one end of the coaxial cable 102 to drive the coaxial cable 102 to slide in the insulating outer sleeve 3, which affects the accuracy of the moving distance of the coaxial cable 102. Therefore, by bonding the coaxial cable 102 to the insulating inner sleeve 104 with a certain rigidity, the overall rigidity is improved and the bending of the coaxial cable 102 is not easy to occur. In addition, the smoothness of the outer surface of the insulating inner sleeve 104 also reduces the friction coefficient between the electrode core wire 1 and the insulating outer sleeve 3, thus making it easier for the coaxial cable 102 to slide in the insulating outer sleeve 3.

In order to ensure that the high-frequency energy can be concentrated at a minimum point of the electrode tip to successfully perform the surgical operations of coagulation and incision of the tissue, the electrode tip 101 may be configured as a needle-shaped structure, a column-shaped structure, a sheet-shaped structure, or a structure of the aforementioned various shapes with a certain bending angle. When used only for tissue coagulation, the electrode tip 101 may also be configured as a spherical structure.

Since the axial part of the coaxial cable 102 is made up of metal and is generally a copper wire, in order to ensure smooth progress of subsequent surgical operations such as cutting and coagulation, the material of the electrode tip 101 may be stainless steel, tungsten wire, and etc., and the end thereof may be polished to avoid mechanical damage to tissues caused by burrs and sharper parts. The surface of the electrode tip 101 can be plated with gold or silver to prevent tissue adhesion.

In order to avoid the unexpected high-frequency current flowing through the human body during the surgery and damaging the human body, and to enable the electrode tip 101 to perform various operations such as cutting, coagulation, and punching and fistulation, the cable of the surgical electrode assembly may be the coaxial cable 102 which includes a center conductor 1021, and an insulating layer 1022, a conducting layer 1023, and an outer insulating skin 1024 that sequentially sleeve on the center conductor 1021 from inside to outside, as shown in FIG. 1. The center conductor 1021 is used for connection with the electrode tip 101. An end portion of the center conductor 1021 and an end portion of the insulating layer 1022 are located inside the electrode insulating tube 103, and the conducting layer 1023 and the outer insulating skin 1024 are located outside the electrode insulating tube 103. When the electrode tip 101 is connected to the coaxial cable 102, a portion of the outer insulating skin 1024 and a portion of the conducting layer 1023, which extend from an end of the coaxial cable 102 and have a length of 4mm to 10 mm, are removed without removing the center conductor 1021 and the insulating layer 1022. The electrode tip 101 is connected to the center conductor 1021 by means of pressure bonding or soldering.

By providing the insulating layer 1022, locating an end portion of the center conductor 1021 and an end portion of the insulating layer 1022 inside the electrode insulating tube 103, and locating the conducting layer 1023 and the outer insulating skin 1024 outside the electrode insulating tube 103, the insulating effect of the overall coaxial cable 102 is ensured.

The center conductor 1021, the electrode tip 101, the human tissue, the insulating outer sleeve 3, the insulating inner sleeve 104, the outer insulating skin 1024, and the conducting layer 1023 form a circuit, which prevents high-frequency current from directly flowing through the human body and damaging the human body when the negative electrode is attached to the human skin. Also, in the circuit, only the portion of the electrode tip 101 that contacts the human skin generates heat, so the contact area is small, and the functions of cutting, coagulation, and punching and fistulation can be realized. The conducting layer 1023 may be in the form of a mesh or a metal film.

The characteristic impedance of the coaxial cable 102 may be greater than that of the output cable of the external host (a host of an electromagnetic knife operation system). The equivalent load impedance of the electrode assembly of the present disclosure is determined by the physical structure and insulation distance between the electrode tip 101 and the conducting layer 1023 of the coaxial cable 102. The physical structure generally includes physiological saline, the wall of the insulating outer sleeve 3, and the insulating sheath of the coaxial cable, etc. The equivalent load impedance of the electrode assembly can be obtained by actual measurement and is generally greater than the characteristic impedance of the coaxial cable 102. By matching the equivalent loads from the host of the electromagnetic knife operation system to the electrode assembly for endoscopic surgery, a high-frequency electrical signal can be effectively transmitted between the electrode tip 101 and the conducting layer 1023, and the reflection of the high-frequency electrical signal between various transmission media can be reduced, so as to achieve the condition of generating near-field electromagnetic field effect between the electrode tip 101 and the tissue, thus achieving the surgery purpose of vaporising or cutting the tissue.

In the present disclosure, high-frequency current (200 kHz to 30 MHz) is used for operation. Therefore, by using the coaxial cable 102 described above, the loss of high-frequency signal during transmission is reduced, and the smooth and accurate operation of the subsequent operation can be ensured.

To facilitate the control of the telescoping of the electrode tip 101, as shown in FIG. 2, the electrode core wire 1 further includes a coaxial connector 105. The telescopic control member 2 includes a tubular body 201, a movable member 202, an end cap 203, and a fixed tube 204. The movable member 202 axially movably sleeves on one end of the tubular body 201. The end cap 203 is fixed to the other end of the tubular body 201 and is connected to the coaxial connector 105. The coaxial connector 105 and the fixed tube 204 are disposed inside the tubular body 201, and the coaxial cable 102 passes through the movable member 202 and the fixed tube 204, and thereafter is electrically coupled to the coaxial connector 105 and the output end of the external host. The movable member 202 is connected to the insulating outer sleeve 3.

When it is necessary to control the electrode tip 101 to extend out of the insulating outer sleeve 3 or retract into the insulating outer sleeve 3, it is only necessary to control the movable member 202 to move axially along the tubular body 201, which in turn drives the insulating outer sleeve 3 to move, such that the electrode tip 101 extends out of the insulating outer sleeve 3 or retracts into the insulating outer sleeve 3. When a surgery is required, an external host is used to transmit electric energy to the electrode tip 101 sequentially through the coaxial connector 105 and the coaxial cable 102 fixed on the end cap 203, so as to control the electrode tip 101 to operate.

The fixed tube 204 may be a metal tube or other tube with sufficient rigidity to prevent the coaxial cable 102 inside the fixed tube 204 from bending during operation.

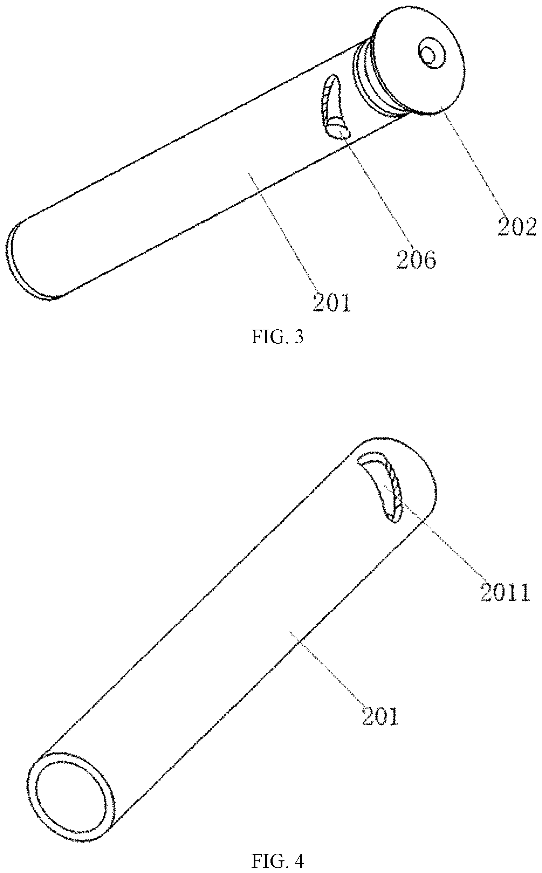

Further, the axial movement of the movable member 202 along the tubular body 201 may be achieved in various ways. As a first example, as shown in FIGS. 3, 4 and 5, the telescopic control member 2 further includes a locating pin 206. The movable member 202 is provided with a locating hole 2021 for fixing the locating pin 206. The tubular body 201 is provided with a guiding groove 2011 in which the locating pin 206 is slidably provided.

By this arrangement, when it is necessary to control the electrode tip 101 to extend out of the insulating outer sleeve 3 or retract into the insulating outer sleeve 3, it is only necessary to rotate the movable member 202 to move the locating pin 206 along the guiding groove 2011. In this process, the telescoping distance of the electrode tip 101 can be controlled according to the moving distance of the locating pin 206 in the guiding groove.

The structure of the guiding groove 2011 may be various. For example, the guiding groove 2011 may be of arc shape or strip shape. The arrangement direction of the guiding groove 2011 may be consistent with the axial direction of the tubular body 201, or may be inclined at a predetermined angle relative to the axial direction of the tubular body 201, as long as enabling the movable member 202 to move in the axial direction. For example, the predetermined angle may be 45 degrees or 60 degrees. In addition, as shown in FIG. 4, a zigzag structure may be provided on the guiding groove 2011 to indicate that the movable member 202 has moved a preset distance. When the locating pin 206 passes through the apex of the zigzag structure, the hand of an operator holding the movable member 202 may have a position sensation like "clicking, clicking and clicking", so as to indicate to the operator how far the electrode tip 101 extends or retracts.

As a second example, the movable member 202 may be threadedly engaged with the tubular body 201. Specifically, an internal thread may be provided on the inner surface of the tubular body 201, and an external thread adapted to the internally threaded hole may be provided on the outer surface of the movable member 202, so that it is only necessary to rotate the movable member 202 during operation.

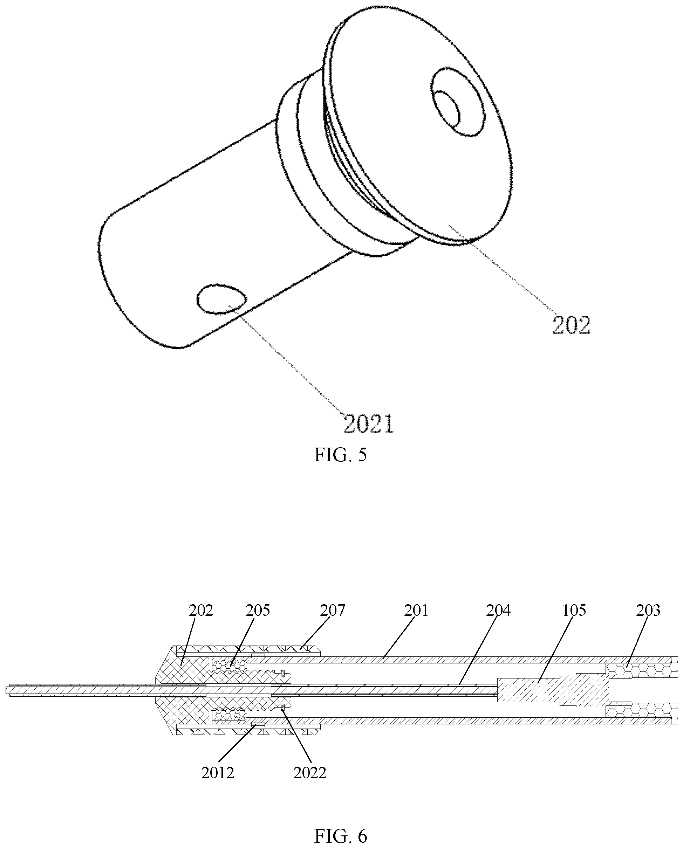

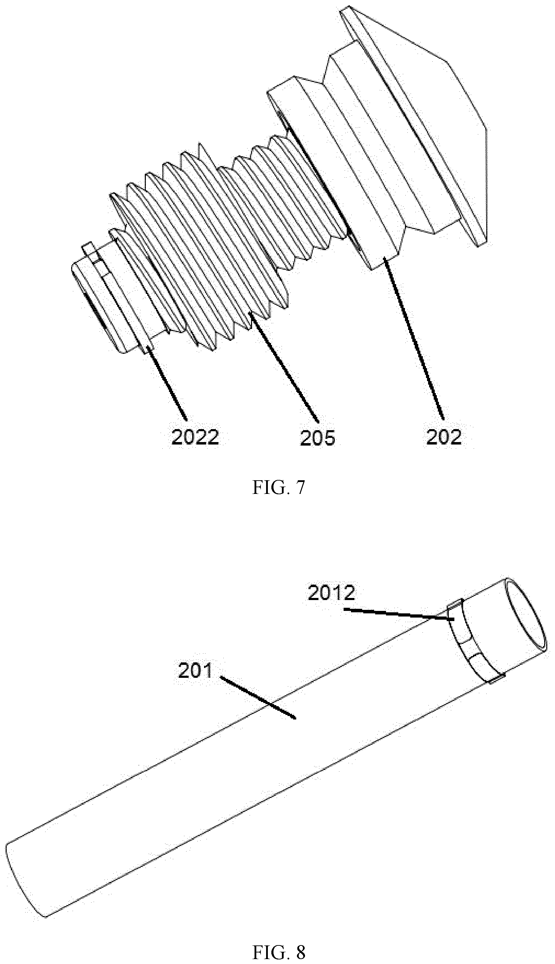

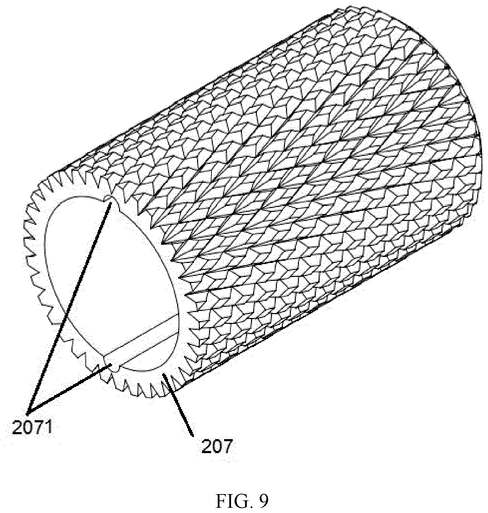

Furthermore, as shown in FIGS. 6 and 7, the telescopic control member 2 further includes a threaded connecting ring 205, an internal thread of which is connected with the external thread of the movable member 202, and an external thread of which is connected with and bonded to the internal thread of the tubular body 201. Referring to FIGS. 6 and 7, the movable member 202 is provided with a limiting snap ring 2022 for restricting the upward moving position of the movable member 202. Referring to FIGS. 6 and 8, the outer surface of the tubular body 201 is provided with an annular groove for installing a position indicating reed 2012. The reed 2012 is provided with two resilient projections at an interval of 180°. The movable member 202 further includes a fixed cylinder 207 bonded to the movable member 202. Referring to FIG. 9, the inner surface of the fixed cylinder 207 is provided with two longitudinal grooves 2071 at an interval of 180° for matching with the resilient projections of the reed 2012, so as to provide a kind of positioning that can be perceived by hand.

By this arrangement, when it is necessary to control the electrode tip 101 to extend out of the insulating outer sleeve 3 or retract into the insulating outer sleeve 3, it is only necessary to rotate the fixed cylinder 207 of the movable member 202 by hand, so that the external thread of the movable member 202 moves along the internal thread of the threaded connecting ring 205 fixed to the tubular body 201, thus achieving the longitudinal movement of the movable member 202. In this process, the telescoping distance of the electrode tip 101 is controlled according to the moving distance of the movable member 202 in the threaded connecting ring 205. When the fixed cylinder 207 is rotated, the resilient projections on the reed 2012 on the tubular body 201 enters into the longitudinal groove on the inner surface of the fixed cylinder 207 every rotation by 180°, so that the operator can feel the change of the rotational force, which reminds the operator that the electrode tip 101 has been extended or retracted by a certain distance.

Since the distance between the electrode tip 101 and the electrode insulating tube 103 is relatively large, in order to avoid shaking of the electrode tip 101 and ensure that the electrode tip 101 has better insulating performance and sealing performance, a sealing member 4 may be filled between the electrode tip 101 and the electrode insulating tube 103, as illustrated in FIG. 1.

The insulating inner sleeve 104 and the insulating outer sleeve 3, which need to have excellent electrical insulation, wear resistance, smoothness, and physical and mechanical strength, may be made up of polyimide, teflon, etc.

The coaxial cable 102, the electrode insulating tube 103, the insulating inner sleeve 104, and the insulating outer sleeve 3 may be integrally molded to ensure a reliable sealing and a tight connection between any two of them.

In conclusion, in the electrode assembly for endoscopic surgery provided by the embodiments of the present disclosure, the electrode tip 101 is configured as needle shape, which ensures that the high-frequency energy can be concentrated at a minimum point of the electrode tip 101, thus enabling the successful surgical operation of coagulation or even incision of the tissue. The electrode insulating tube 103 and the insulating inner sleeve 104 sleeve on the outside of the electrode tip 101, which improves the insulating property of the head portion of the electrode tip 101, avoids high-frequency leakage of the electrode tip 101 in the perfusion liquid, avoids the weakening of the electric field strength of the head portion and the inability to achieve the field intensity required for coagulation or incision using the near field effect caused by the high-frequency leakage, and reduces the damage to the patient caused by current leakage, such that the surgery is safer and more effective and the operations of vaporization and incision can be performed at a very low power. The telescopic control member 2 and the insulating outer sleeve 3 are provided such that the insulating outer sleeve 3 movably sleeves on the outside of the electrode insulating tube 103. One end of the insulating outer sleeve 3 where the electrode tip 101 is located is a preset distance longer than the electrode insulating tube 103, and the movement of the insulating outer sleeve 3 is controlled by the telescopic control member to retract the electrode tip 101 into the electrode insulating tube 3, which ensures that the electrode tip 101 can be retracted into the insulating outer sleeve 3 during passing through the endoscope channel and can extend out of the insulating outer sleeve 3 after entry into the lesion area to perform the subsequent surgical operations, thereby preventing the electrode tip 101 from scratching the endoscope channel while passing through the endoscope channel, and avoiding affecting the service life of the endoscope.

The purpose, technical features and technical effects of the present disclosure have been further described above by means of some embodiments. It should be understood that the embodiments are meant to facilitate understanding of the principles of the present disclosure, rather than limit the scope of the present disclosure. Any modifications, alternations, improvements, etc., made by those skilled in the art without departing from the concepts and principles of this disclosure shall fall within the scope of the present disclosure.

## Claims

1. An electrode assembly for endoscopic surgery, comprising an electrode core wire (1), a telescopic control member (2), and an insulating outer sleeve (3), wherein
the electrode core wire (1) comprises an electrode tip (101), a coaxial cable (102), and an electrode insulating tube (103), wherein the electrode tip (101) is electrically coupled to an output end of an external host through the coaxial cable (102), and the electrode insulating tube (103) sleeves on the electrode tip (101);
the insulating outer sleeve (3) movably sleeves on the outside of the electrode core wire (1), and one end of the insulating outer sleeve (3) where the electrode tip (101) is located is a preset distance longer than the electrode insulating tube (103);
the telescopic control member (2) is configured to control the movement of the insulating outer sleeve (3) so that the electrode tip (101) extends out of the insulating outer sleeve (3) or retracts into the insulating outer sleeve (3).

2. The electrode assembly for endoscopic surgery according to claim 1, wherein the electrode tip (101) is of needle shape.

3. The electrode assembly for endoscopic surgery according to claim 1, wherein the coaxial cable (102) comprises a center conductor (1021), and an insulating layer (1022), a conducting layer (1023), and an outer insulating skin (1024) that sequentially sleeve on the center conductor (1021) from inside to outside;
the center conductor (1021) is configured to connect with the electrode tip (101);
an end portion of the center conductor (1021) and an end portion of the insulating layer (1022) are located inside the electrode insulating tube (103), and the conducting layer (1023) and the outer insulating skin (1024) are located outside the electrode insulating tube (103).

4. The electrode assembly for endoscopic surgery according to claim 3, wherein the electrode core wire (1) further comprises an insulating inner sleeve (104) which seals and sleeves on the outside of the electrode insulating tube (103) and the outside of the outer insulating skin (1024);
the insulating outer sleeve (3) slidably sleeves on the outside of the insulating inner sleeve (104).

5. The electrode assembly for endoscopic surgery according to claim 1, wherein the electrode core wire (1) further comprises a coaxial connector (105);
the telescopic control member (2) comprises a tubular body (201), a movable member (202), an end cap (203), and a fixed tube (204);
the movable member (202) axially movably sleeves on an end portion of the tubular body (201);
the end cap (203) is fixed to the other end portion of the tubular body (201) and is connected to the coaxial connector (105);
the coaxial connector (105) and the fixed tube (204) are disposed inside the tubular body (201);
the coaxial cable (102) passes through the movable member (202) and the fixed tube (204), and thereafter is electrically coupled to the coaxial connector (105) and the output end of the external host;
the movable member (202) is connected to the insulating outer sleeve (3).

6. The electrode assembly for endoscopic surgery according to claim 5, wherein the telescopic control member (2) further comprises a locating pin (206);
the movable member (202) is provided with a locating hole (2021) for fixing the locating pin (206);
the tubular body (201) is provided with a guiding groove (2011) in which the locating pin (206) is slidably provided.

7. The electrode assembly for endoscopic surgery according to claim 5, wherein the movable member (202) is threadedly engaged with the tubular body (201).

8. The electrode assembly for endoscopic surgery according to claim 4, wherein the insulating inner sleeve (104) and the insulating outer sleeve (3) are smooth sleeves with preset flexibility and rigidity and made up of organic polymer material.

9. The electrode assembly for endoscopic surgery according to claim 4, wherein the coaxial cable (102), the electrode insulating tube (103), the insulating inner sleeve (104), and the insulating outer sleeve (3) are integrally molded.

10. The electrode assembly for endoscopic surgery according to claim 1, wherein a sealing member (4) is filled between the electrode tip (101) and the electrode insulating tube (103), between the insulating inner sleeve (104) and the coaxial cable (102), and between the electrode insulating tube (103) and the coaxial cable (102).
